(19) Europäisches Patentamt
European Patent Office
Office européen des brevets

(11) Veröffentlichungsnummer: **0 384 244 A1**

# (12) EUROPÄISCHE PATENTANMELDUNG

(21) Anmeldenummer: **90102636.9**

(22) Anmeldetag: **10.02.90**

(51) Int. Cl.[5]: **C07D 413/04, C07D 417/04, C07D 413/14, C07D 417/14, C07D 277/52, C07D 263/48, C07D 285/08, C07D 285/12, C07D 271/07, C07D 271/113, A01N 43/76**

(30) Priorität: **24.02.89 DE 3905714**

(43) Veröffentlichungstag der Anmeldung:
**29.08.90 Patentblatt 90/35**

(84) Benannte Vertragsstaaten:
**BE CH DE DK ES FR GB IT LI NL SE**

(71) Anmelder: **BAYER AG**

**D-5090 Leverkusen 1 Bayerwerk(DE)**

(72) Erfinder: **Wolf, Hilmar**
**Haus Gravener Strasse 105**
**D-4018 Langenfeld(DE)**
Erfinder: **Wroblowsky, Heinz-Jürgen**
**Gladbacher Strasse 34**
**D-4018 Langenfeld(DE)**
Erfinder: **Babczinski, Peter**
**In der Lohrenbeck 11**
**D-5600 Wuppertal 1(DE)**
Erfinder: **Lürssen, Klaus**
**August-Kierspel-Strasse 145**
**D-5060 Bergisch-Gladbach 2(DE)**
Erfinder: **Santel, Hans-Joachim**
**Grünstrasse 9a**
**D-5090 Leverkusen 1(DE)**
Erfinder: **Schmidt, Robert R.**
**Im Waldwinkel 110**
**D-5060 Bergisch-Gladbach 2(DE)**

(54) **Sulfonylimino-azinylheteroazole, Verfahren und Zwischenprodukte zu ihrer Herstellung und ihre Verwendung als Herbizide.**

(57) Die Erfindung betrifft neuartige Sulfonylimino-azinylheteroazole der allgemeinen Formel

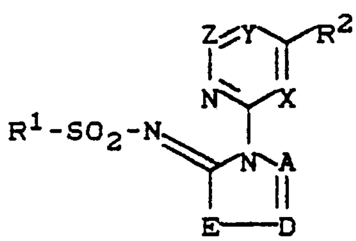

(I)

in welcher
A für Stickstoff oder die Gruppierung C-$A^1$ steht,
D für Stickstoff oder die Gruppierung C-$D^1$ steht,
(mit der Maßgabe, daß A und D nicht beide gleichzeitig für Stickstoff stehen,)
E für Sauerstoff oder Schwefel steht,
X für Stickstoff oder eine CH-Gruppierung steht,
Y für Stickstoff oder die Gruppierung C-$R^3$ steht und

EP 0 384 244 A1

Z für Stickstoff oder die Gruppierung C-$R^4$ steht,
(wobei die Reste $A^1$, $D^1$, $R^1$, $R^2$, $R^3$ und $R^4$ die in der Beschreibung angegebenen Bedeutungen haben,)
ein Verfahren und neue Zwischenprodukte zu ihrer Herstellung sowie ihre Verwendung als Herbizide.

# Sulfonylimino-azinylheteroazole, Verfahren und Zwischenprodukte zu ihrer Herstellung und ihre Verwendung als Herbizide

Die Erfindung betrifft neuartige Sulfonylimino-azinylheteroazole, ein Verfahren und neue Zwischenprodukte zu ihrer Herstellung sowie ihre Verwendung als Herbizide.

Es ist bekannt, daß 3-Amino-1,2,4-triazol (Amitrol) als Herbizid verwendet werden kann (vgl. Science 145 (1964), 97). Die Wirkung dieser Verbindung ist jedoch nicht in allen Belangen zufriedenstellend.

Es wurden nun die neuen Sulfonylimino-azinylheteroazole der allgemeinen Formel (I)

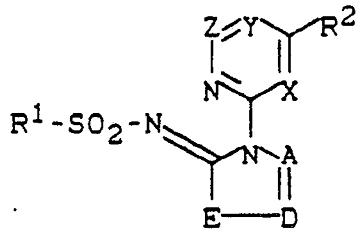

(I)

in welcher

A für Stickstoff oder die Gruppierung C-$A^1$ steht, worin

$A^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,

D für Stickstoff oder die Gruppierung C-$D^1$ steht, worin

$D^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,

(mit der Maßgabe, daß nicht A und D beide gleichzeitig für Stickstoff stehen),

E für Sauerstoff oder Schwefel steht,

$R^1$ für jeweils gegebenenfalls substituiertes Alkyl, Aralkyl, Aryl oder Heteroaryl steht,

$R^2$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht,

X für Stickstoff oder eine CH-Gruppierung steht,

Y für Stickstoff oder die Gruppierung C-$R^3$ steht, worin

$R^3$ für Wasserstoff, Halogen, Cyano, Alkyl, Formyl, Alkylcarbonyl oder Alkoxycarbonyl steht, und

Z für Stickstoff oder die Gruppierung C-$R^4$ steht, worin

$R^4$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht,

gefunden.

Man erhält die neuen Sulfonylimino-azinylheteroazole der allgemeinen Formel (I), wenn man Sulfonylaminoheteroazole der allgemeinen Formel (II)

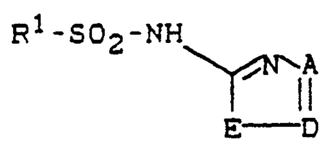

(II)

in welcher

A, D, E und $R^1$ die oben angegebenen Bedeutungen haben,

mit Azinen der allgemeinen Formel (III)

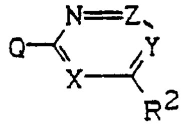

(III)

in welcher

$R^2$, X, Y und Z die oben angegebenen Bedeutungen haben und
Q für Halogen oder Alkylsulfonyl steht,
gegebenenfalls in Gegenwart eines Säureakzeptors und gegebenenfalls in Gegenwart eines Verdünnungsmittels umsetzt.

Die neuen Sulfonylimino-azinylheteroazole der allgemeinen Formel (I) zeichnen sich durch starke herbizide Wirksamkeit aus. Diese Verbindungen stellen eine chemisch neuartige Klasse von Herbiziden dar.

Überraschenderweise zeigen die neuen Verbindungen der Formel (I) erheblich bessere herbizide Wirkung als das strukturell ähnliche bekannte Herbizid Aminotriazol (Amitrol).

Gegenstand der Erfindung sind vorzugsweise Verbindungen der Formel (I), in welcher
A für Stickstoff oder die Gruppierung C-$A^1$ steht, worin
$A^1$ für Wasserstoff, Fluor, Chlor, Brom, Iod, Cyano, Nitro oder für jeweils gegebenenfalls durch Fluor und/oder Chlor oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkylamino oder Di-($C_1$-$C_4$)-alkyl-amino oder für gegebenenfalls durch Fluor, Chlor, Brom, $C_1$-$C_4$-Alkyl, $C_1$-$C_2$-Fluor-und/oder -Chlor-alkyl, $C_1$-$C_4$-Alkoxy und/oder $C_1$-$C_2$-Fluor- und/oder Chlor-alkoxy substituiertes Phenyl steht,
D für Stickstoff oder die Gruppierung C-$D^1$ steht, worin
$D^1$ für Wasserstoff, Fluor, Chlor, Brom, Iod, Cyano, Nitro oder für jeweils gegebenenfalls durch Fluor und/oder Chlor oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkylamino oder Di-($C_1$-$C_4$)-alkylamino oder für gegebenenfalls durch Fluor, Chlor, Brom, $C_1$-$C_4$-Alkyl, $C_1$-$C_2$-Fluor-und/oder Chlor-alkyl, $C_1$-$C_4$-Alkoxy und/oder $C_1$-$C_2$-Fluor- und/oder Chlor-alkoxy substituiertes Phenyl steht,
(mit der Maßgabe, daß nicht A und D beide gleichzeitig für Stickstoff stehen),
E für Sauerstoff oder Schwefel steht,
$R^1$ für den Rest

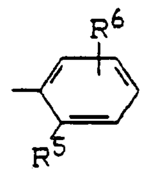

steht, worin
$R^5$ und $R^6$ gleich oder verschieden sind und für Wasserstoff, Fluor, Chlor, Brom, Iod, Cyano, Nitro, $C_1$-$C_6$-Alkyl [welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-al kyl)-aminosulfonyl, $C_3$-$C_6$-Cylcoalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio [welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], für $C_3$-$C_6$-Alkenyloxy [welches gegebenenfalls durch Fluor, Chlor, Brom, Cyano oder $C_1$-$C_4$-Cyano oder $C_1$-$C_4$-Alkoxy-carbonyl substituiert ist], für $C_2$-$C_6$-Alkenylthio [welches gegebenenfalls durch Fluor, Chlor, Brom, Cyano, Nitro, $C_1$-$C_3$-Alkylthio oder $C_1$-$C_4$-Alkoxy-carbonyl substituiert ist], $C_3$-$C_6$-Alkinyloxy, $C_3$-$C_6$-Alkinylthio oder für den Rest $-S(O)_p-R^7$ stehen, wobei
p für die Zahlen 1 oder 2 steht und
$R^7$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^8$ steht, wobei
$R^8$ für $C_1$-$C_6$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist], für Benzhydryl oder für Phenyl [welches gegebenenfalls durch

Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht,

$R^5$ und $R^6$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonylamino, Di-($C_1$-$C_4$-alkyl)-amino-carbonylamino, oder für den Rest -CO-$R^9$ stehen, wobei

$R^9$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

$R^5$ und $R^6$ weiterhin für $C_1$-$C_4$-Alkylsulfonyloxy, Di-($C_1$-$C_4$-alkyl)-aminosulfonylamino oder für den Rest -CH=N-$R^{10}$ stehen, wobei

$R^{10}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/ oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Ci-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

worin weiter

$R^1$ für den Rest

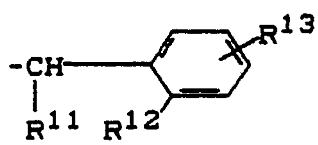

steht, worin

$R^{11}$ für Wasserstoff oder $C_1$-$C_4$-Alkyl steht,

$R^{12}$ und $R^{13}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; worin weiter

$R^1$ für den Rest

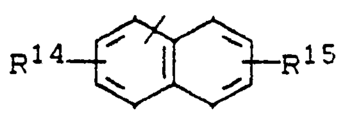

steht, worin

$R^{14}$ und $R^{15}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; worin weiter

$R^1$ für den Rest

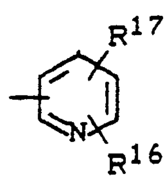

steht, worin

$R^{16}$ und $R^{17}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_2$-$C_4$-Alkenyl [welches gegebenenfalls durch Fluor und/oder Chlor substituiert ist], $C_1$-$C_4$-Alkoxy [welches gegebenenfalls durch Fluor

und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di($C_1$-$C_4$-alkyl)-aminosulfonyl, $C_1$-$C_4$-Alkoxy-carbonyl, Dimethylaminocarbonyl oder Dioxolanyl stehen; worin weiter
$R^1$ für den Rest

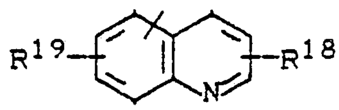

steht, worin
$R^{18}$ und $R^{19}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$- Alkylthio, $C_1$-$C_4$-Alkylsulfinyl oder $C_1$-$C_4$-Alkylsulfonyl [welche gegebenenfalls durch Fluor und/oder Chlor substituiert sind], oder für Di-($C_1$-$C_4$-alkyl)-aminosulfonyl stehen; worin weiter
$R^1$ für den Rest

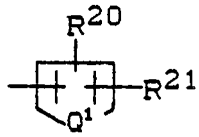

steht, worin
$R^{20}$ und $R^{21}$ gleich oder verschieden sind und für Wasserstoff, Fluor, Chlor, Brom, Cyano, Nitro, $C_1$-$C_4$-Alkyl [welches gegebenenfalls durch Fluor, Chlor, $C_1$-$C_4$-Alkoxy und/oder $C_1$-$C_4$-Halogenalkoxy substituiert ist], $C_1$-$C_4$-Alkoxy [welches gegebenenfalls durch Fluor und/ oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-amino-sulfonyl oder $C_1$-$C_4$-Alkoxy-carbonyl stehen, und
$Q^1$ für Sauerstoff, Schwefel oder die Gruppierung N-$Q^2$ steht, wobei
$Q^2$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcarbonyl, $C_1$-$C_4$-Alkoxy-carbonyl oder Di-($C_1$-$C_4$-alkyl)-aminocarbonyl steht; worin weiter
$R^1$ für den Rest

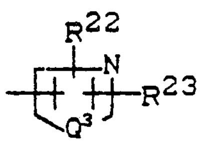

steht, worin
$R^{22}$ und $R^{23}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,
$Q^3$ für Schwefel oder die Gruppierung N-$R^{24}$ steht, wobei
$R^{24}$ für Wasserstoff oder $C_1$-$C_4$-Alkyl steht; worin weiter
$R^1$ für den Rest

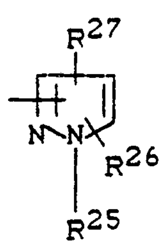

steht, worin
$R^{25}$ für Wasserstoff, $C_1$-$C_4$-Alkyl, Phenyl oder (Iso)Chinolinyl steht,
$R^{26}$ für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Dioxolanyl oder $C_1$-$C_4$-Alkoxy-carbonyl steht und
$R^{27}$ für Wasserstoff, Halogen oder $C_1$-$C_4$-Alkyl steht; worin weiter
$R^1$ für den Rest

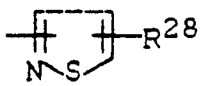

steht, worin
$R^{28}$ für Wasserstoff, Halogen, $C_1$-$C_4$-Alkyl oder $C_1$-$C_4$-Alkoxy-carbonyl steht; worin weiter
$R^1$ für den Rest

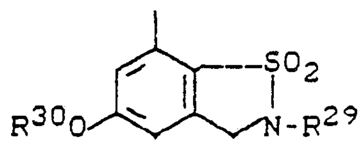

steht, worin
$R^{29}$ für $C_1$-$C_4$-Alkyl steht und
$R^{30}$ für $C_1$-$C_4$-Alkyl steht, worin weiter
$R^1$ für den Rest

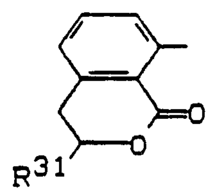

steht, worin
$R^{31}$ für Wasserstoff oder Methyl steht,
$R^2$ für Wasserstoff, Fluor, Chlor, Brom, Hydroxy, Amino, $C_1$-$C_4$-Alkyl, $C_1$-$C_4$-Halogenalkyl, $C_1$-$C_2$-Alkoxy-$C_1$-$C_2$-alkyl, Bis-($C_1$-$C_2$-alkoxy)-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylsulfinyl, $C_1$-$C_4$-Alkylsulfonyl, $C_1$-$C_4$-Alkylamino, Dimethylamino oder Diethylamino steht,
X für Stickstoff oder eine CH-Gruppierung steht,
Y für Stickstoff oder die Gruppierung C-$R^3$ steht, worin
$R^3$ für Wasserstoff, Fluor, Chlor, Brom, Cyano, Methyl, Formyl, Acetyl, Methoxycarbonyl oder Ethoxycarbonyl steht, und
Z für Stickstoff oder die Gruppierung C-$R^4$ steht, worin
$R^4$ für Wasserstoff, Fluor, Chlor, Brom, Hydroxy, Amino, $C_1$-$C_4$-Alkyl, $C_1$-$C_4$-Halogenalkyl, $C_1$-$C_2$-Alkoxy-$C_1$-$C_2$-alkyl, Bis-($C_1$-$C_2$-alkoxy)-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylsulfinyl, $C_1$-$C_4$-Alkylsulfonyl, $C_1$-$C_4$-Alkylamino, Dimethylamino oder Diethylamino steht.

Gegenstand der Erfindung sind insbesondere Verbindungen der Formel (I), in welcher
A für Stickstoff oder die Gruppierung C-$A^1$ steht, worin
$A^1$ für Wasserstoff, Methyl, Ethyl, Propyl, Isopropyl, Butyl, Isobutyl, sec-Butyl, tert-Butyl, Difluormethyl, Trifluormethyl, Dichlormethyl, Trichlormethyl, Chlordifluormethyl, Fluordichlormethyl, Methylthio, Ethylthio, Propylthio, Butylthio oder Phenyl steht,
D für Stickstoff oder die Gruppierung C-$D^1$ steht, worin
$D^1$ für Wasserstoff, Methyl, Ethyl, Propyl, Isopropyl, Butyl, Isobutyl, sec-Butyl, tert-Butyl, Difluormethyl, Trifluormethyl, Dichlormethyl, Trichlormethyl, Chlordifluormethyl, Fluordichlormethyl, Methylthio, Ethylthio, Propylthio, Butylthio oder Phenyl steht,

(mit der Maßgabe, daß nicht A und D beide gleichzeitig für Stickstoff stehen), E für Sauerstoff oder Schwefel steht, $R^1$ für den Rest

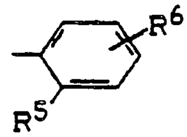

steht worin $R^5$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$- $C_3$-Alkylsulfonyl, Dimethylaminosulfonyl, N-Methoxy-N-methylaminosulfonyl, Phenyl, Phenoxy oder $C_1$-$C_3$-Alkoxy-carbonyl steht und $R^6$ für Wasserstoff, Fluor oder Chlor steht; worin weiter $R^1$ für den Rest

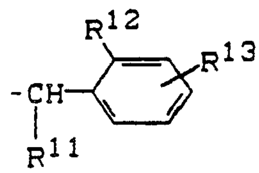

steht, worin $R^{11}$ für Wasserstoff steht, $R^{12}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und $R^{13}$ für Wasserstoff, Fluor oder Chlor steht; worin weiter $R^1$ für den Rest

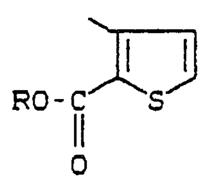

steht, worin R für $C_1$-$C_4$-Alkyl steht, oder $R^1$ für den Rest

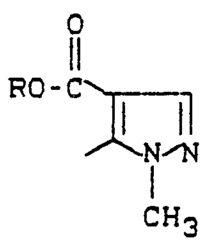

steht, worin R für $C_1$-$C_4$-Alkyl steht, oder $R^1$ für den Rest

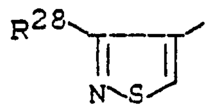

steht, worin

$R^{28}$ für Wasserstoff, Chlor, Methyl, Ethyl, Methoxycarbonyl oder Ethoxycarbonyl steht;

in welcher weiter

$R^2$ für Wasserstoff, Fluor, Chlor, Brom, Methyl, Ethyl, Trifluormethyl, Methoxy, Ethoxy, Difluormethoxy, Methylthio, Ethylthio, Methylamino, Ethylamino oder Dimethylamino steht,

X für Stickstoff oder eine CH-Gruppierung steht,

Y für Stickstoff oder die Gruppierung C-$R^3$ steht, worin

$R^3$ für Wasserstoff, Fluor, Chlor oder Methyl steht und

Z für Stickstoff oder die Gruppierung C-$R^4$ steht, worin

$R^4$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino oder Dimethylamino steht.

Verwendet man beispielsweise 2-(2-Fluor-phenylsulfonylamino)-oxazol und 4,6-Dimethyl-2-methylsulfonylpyrimidin als Ausgangsstoffe, so kann der Reaktionsablauf beim erfindungsgemäßen Verfahren durch das folgende Formelschema skizziert werden:

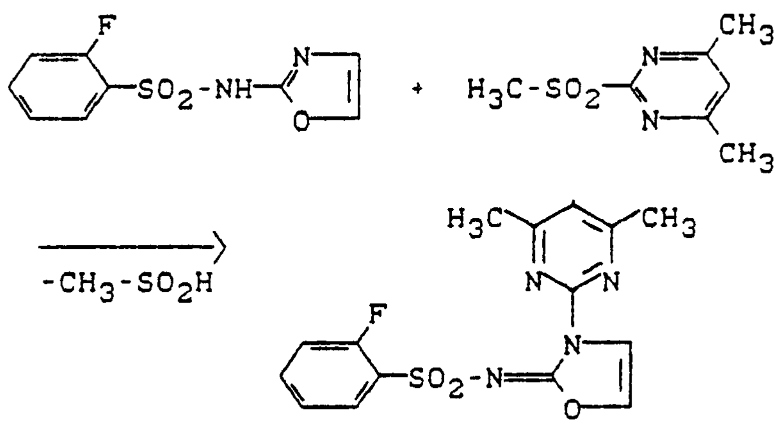


Die beim erfindungsgemäßen Verfahren zur Herstellung von Verbindungen der Formel (I) als Ausgangsstoffe zu verwendenden Sulfonylaminoheteroazole sind durch die Formel (II) allgemein definiert.

In Formel (II) haben A, D, E und $R^1$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, D, E und $R^1$ angegeben wurden.

Beispiele für die Ausgangsstoffe der Formel (II) sind in der nachstehenden Tabelle 1 aufgeführt.

<u>Tabelle 1:</u> Beispiele für die Ausgangsstoffe der Formel (II)

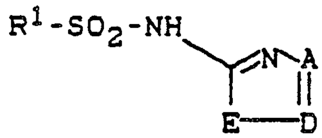

(II)

| A | D | E | $R^1$ |
|---|---|---|---|
| N | CH | O | 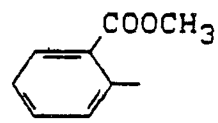 |
| N | CH | S | 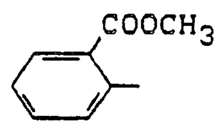 |
| CH | N | O | 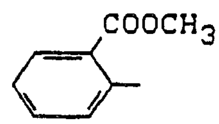 |
| CH | N | S | 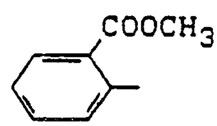 |
| CH | CH | O | 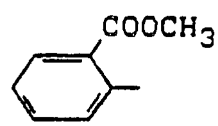 |
| CH | CH | S | 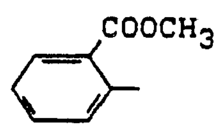 |

Tabelle 1 - Fortsetzung

| A | D | E | $R^1$ |
|---|---|---|---|
| N | $C-CH_3$ | O | 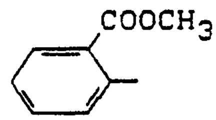 |
| N | $C-CH_3$ | S | 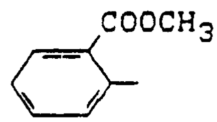 |
| $C-CH_3$ | CH | S | 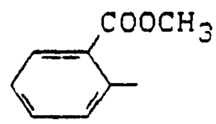 |
| N | $C-CF_3$ | S | 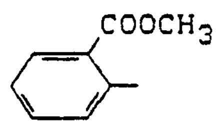 |
| N | $C-C_2H_5$ | S | 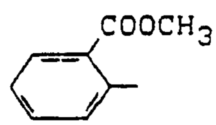 |
| N | $C-C_6H_5$ | S | 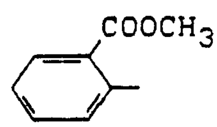 |
| N | $C-SCH_3$ | S | 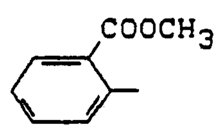 |

Tabelle 1 - Fortsetzung

| A | D | E | $R^1$ |
|---|---|---|---|
| $C-C_2H_5$ | CH | S | 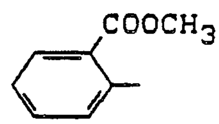 |
| N | $C-CHCl_2$ | S | 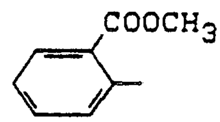 |
| C-Cl | C-Cl | S | 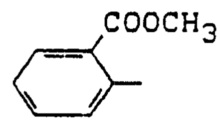 |
| N | CH | O | 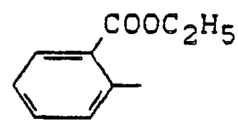 |
| N | CH | S | 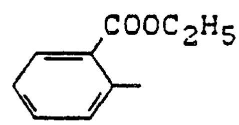 |
| CH | N | S | 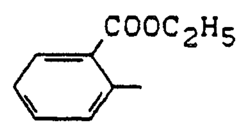 |
| $C-CH_3$ | N | S | 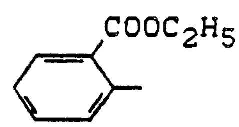 |
| CH | CH | S | 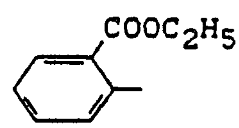 |

Tabelle 1 - Fortsetzung

| A | D | E | $R^1$ |
|---|---|---|---|
| N | $C-CH_3$ | O | 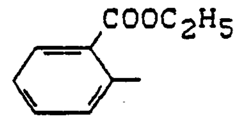 |
| N | $C-CH_3$ | S | 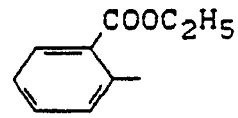 |
| N | $C-SCH_3$ | S | 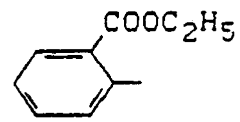 |
| N | CH | S | 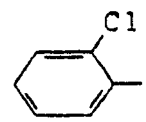 |
| CH | N | S | 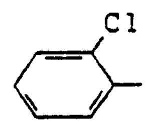 |
| $C-CH_3$ | N | S | 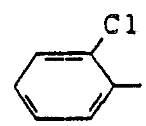 |
| CH | CH | S | 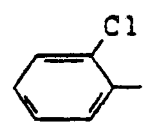 |
| N | $C-CH_3$ | O | 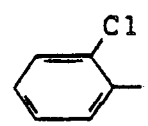 |

Tabelle 1 - Fortsetzung

| A | D | E | $R^1$ |
|---|---|---|---|
| N | $C-CH_3$ | S | 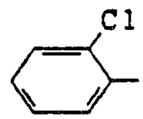 |
| N | $C-CF_3$ | S | 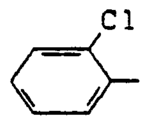 |
| N | $C-SCH_3$ | S | 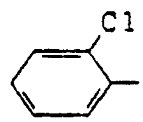 |
| N | $C-C_6H_5$ | S | 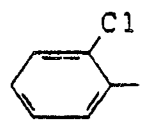 |
| N | $C-CH(CH_3)_2$ | S | 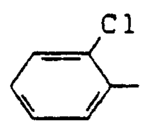 |
| N | $C-SC_4H_9$ | S | 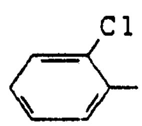 |
| C-Cl | C-Cl | S | 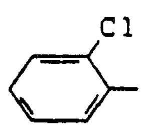 |
| N | CH | S | 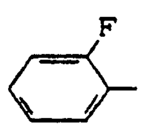 |

Tabelle 1 - Fortsetzung

| A | D | E | $R^1$ |
|---|---|---|---|
| CH | N | S | 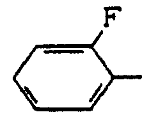 |
| C-$CH_3$ | N | S | 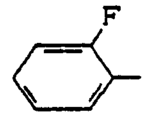 |
| CH | CH | S | 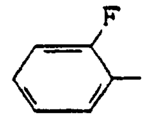 |
| N | C-$CH_3$ | S | 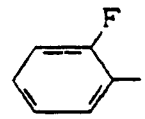 |
| N | C-$SCH_3$ | S | 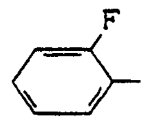 |
| N | CH | S | 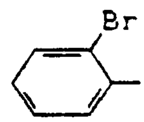 |
| CH | N | S | 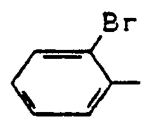 |
| C-$CH_3$ | N | S | 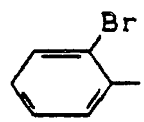 |

Tabelle 1 - Fortsetzung

| A | D | E | $R^1$ |
|---|---|---|---|
| CH | CH | S | 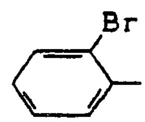 |
| N | $C-CH_3$ | S | 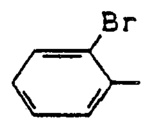 |
| N | CH | S | 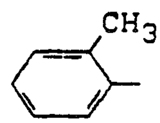 |
| CH | N | S | 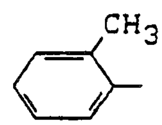 |
| $C-CH_3$ | N | S | 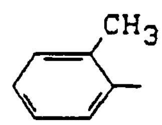 |
| N | $C-CH_3$ | S | 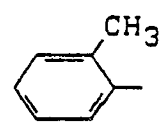 |
| CH | CH | S | 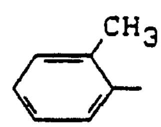 |
| N | $C-SCH_3$ | S | 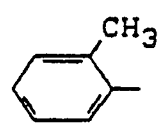 |

Tabelle 1 - Fortsetzung

| A | D | E | $R^1$ |
|---|---|---|---|
| N | CH | S | 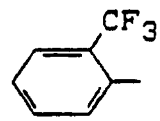 |
| CH | N | S | 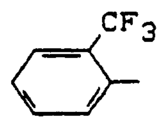 |
| $C-CH_3$ | N | S | 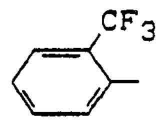 |
| N | $C-CH_3$ | S | 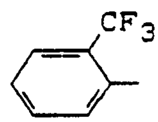 |
| N | CH | S | 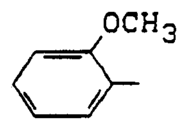 |
| CH | N | S | 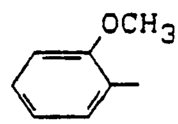 |
| $C-CH_3$ | N | S | 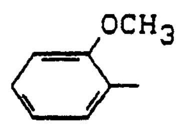 |
| N | $C-CH_3$ | S | 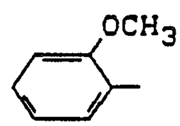 |

Tabelle 1 - Fortsetzung

| A | D | E | $R^1$ |
|---|---|---|---|
| CH | CH | S | 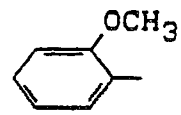 |
| N | $C-SCH_3$ | S | 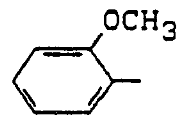 |
| N | $C-C_6H_5$ | S | 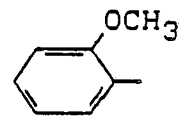 |
| N | CH | S | 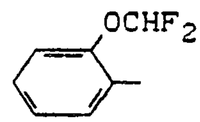 |
| CH | N | S | 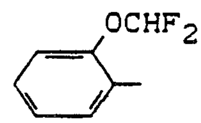 |
| $C-CH_3$ | N | S | 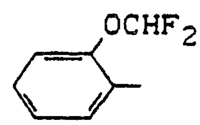 |
| N | $C-CH_3$ | S | 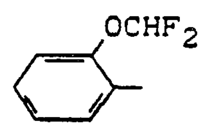 |
| CH | CH | S | 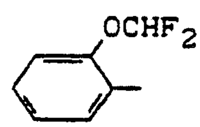 |

Tabelle 1 - Fortsetzung

| A | D | E | $R^1$ |
|---|---|---|---|
| N | $C-SCH_3$ | S | 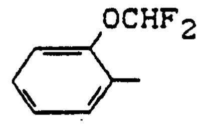 |
| N | $C-C_6H_5$ | S | 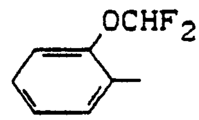 |
| C-Cl | C-Cl | S | 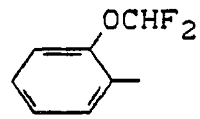 |
| N | CH | S | 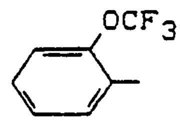 |
| CH | N | S | 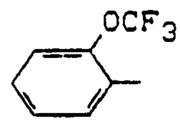 |
| $C-CH_3$ | N | S | 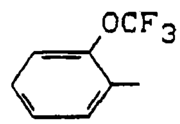 |
| N | $C-CH_3$ | S | 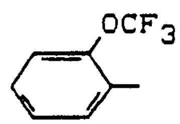 |
| CH | CH | S | 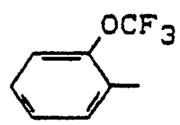 |

Tabelle 1 - Fortsetzung

| A | D | E | $R^1$ |
|---|---|---|---|
| C-Cl | C-Cl | S | 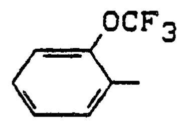 |
| N | CH | S | 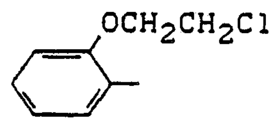 |
| CH | N | S | 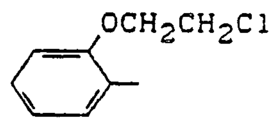 |
| $C-CH_3$ | N | S | 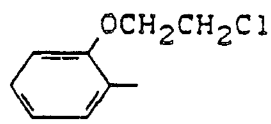 |
| N | $C-CH_3$ | S | 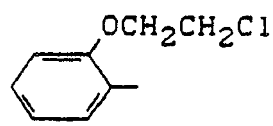 |
| CH | CH | S | 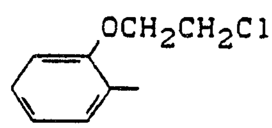 |
| N | $C-SCH_3$ | S | 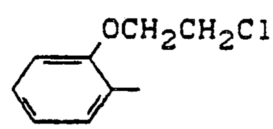 |
| N | $C-CF_3$ | S | 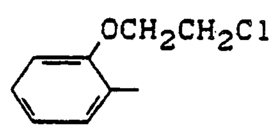 |

<u>Tabelle 1</u> - Fortsetzung

| A | D | E | $R^1$ |
|---|---|---|---|
| N | $C-C_6H_5$ | S | 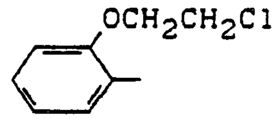 |
| N | CH | S | 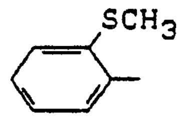 |
| CH | N | S | 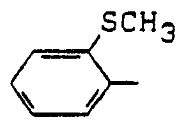 |
| $C-CH_3$ | N | S | 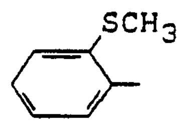 |
| N | $C-CH_3$ | S | 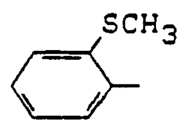 |
| N | $C-SCH_3$ | S | 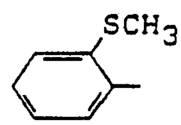 |
| N | $C-CF_3$ | S | 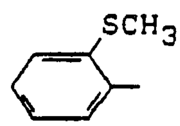 |
| N | $C-C_6H_5$ | S | 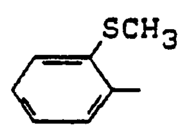 |

Tabelle 1 - Fortsetzung

| A | D | E | $R^1$ |
|---|---|---|---|
| CH | CH | S | 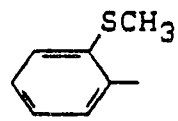 |
| C-Cl | C-Cl | S | 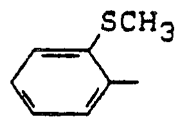 |
| N | CH | S | 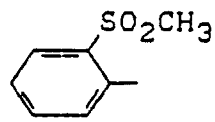 |
| N | CH | S | 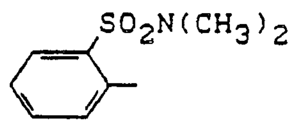 |
| N | CH | S | 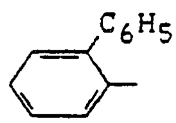 |

Die Ausgangsstoffe der Formel (II) sind noch nicht aus der Literatur bekannt.

Man erhält die neuen Verbindungen der Formel (II) wenn man

α) Aminoheteroazole der allgemeinen Formel (IV)

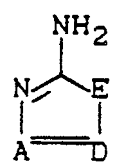

(IV)

in welcher

A, D und E die oben angegebenen Bedeutungen haben,

mit Sulfonsäurehalogeniden oder Sulfonsäureanhydriden der allgemeinen Formel (V)

$R^1$-$SO_2$-$Q^4$ (V)

in welcher

$R^1$ die oben angegebene Bedeutung hat und

$Q^4$ für Chlor, Brom oder die Gruppierung -O-$SO_2$-$R^1$ steht, worin $R^1$ die oben angegebene Bedeutung hat, gegebenenfalls in Gegenwart eines Säureakzeptors, wie z.B. Pyridin und gegebenenfalls in Gegenwart eines Verdünnungsmittels, wie z.B. Pyridin, Methylenchlorid, Aceton, Acetonitril, Tetrahydrofuran, Dioxan oder Dimethylformamid, bei Temperaturen zwischen - 50 °C und + 50 °C umsetzt und gegebenenfalls das Reaktionsprodukt mit Ammoniak behandelt, oder wenn man

β) Heteroazole der allgemeinen Formel (VI)

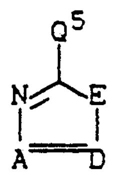

(VI)

in welcher
A, D und E die oben angegebenen Bedeutungen haben und
$Q^5$ für Halogen oder Alkylsulfonyl steht,
mit Sulfonsäureamiden der allgemeinen Formel (VII)
$R^1$-$SO_2$-$NH_2$ (VII)
in welcher
$R^1$ die oben angegebene Bedeutung hat,
gegebenenfalls in Gegenwart eines Säureakzeptors, wie z.B. Kaliumcarbonat, und gegebenenfalls in Gegenwart eines Verdünnungsmittels, wie z.B. Acetonitril, Tetrahydrofuran, Dioxan oder Dimethylformamid, bei Temperaturen zwischen 0 °C und 150 °C umsetzt.

Die als Zwischenprodukte benötigten Aminoheteroazole sind durch die Formel (IV) allgemeinen definiert. In Formel (IV) haben A, D und E vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, D und E angegeben wurden.

Als Beispiele für die Verbindungen der Formel (IV) seien genannt:
2-Amino-oxazol, 2-Amino-thiazol, 2-Amino-1,3,4-thiadiazol, 5-Amino-1,2,4-thiadiazol, 2-Amino-1,3,4-oxadiazol, 2-Amino-4-methyl-thiazol, 2-Amino-5-methylthiazol, 2-Amino-5-methyl-1,3,4-thiadiazol, 2-Amino-5-ethyl-1,3,4-thiadiazol, 2-Amino-5-trifluormethyl-1,3,4-thiadiazol, 2-Amino-5-difluormethyl-1,3,4-thiadiazol, 2-Amino-5-dichlormethyl-1,3,4-thiadiazol, 2-Amino-5-methylthio-1,3,4-thiadiazol und 2-Amino-5-phenyl-1,3,4-thiadiazol.

Die Aminoheteroazole der Formel (IV) sind bekannt und/oder können nach an sich bekannten Verfahren hergestellt werden (vgl. Comprehensive Heterocyclic Chemistry, Vol. 6, Pergamon Press 1984).

Die weiter als Zwischenprodukte zu verwendenden Sulfonsäurehalogenide bzw. -anhydride sind durch die Formel (V) allgemein definiert. In Formel (V) hat $R^1$ vorzugsweise bzw. insbesondere diejenige Bedeutung, die oben im Rahmen der Beschreibung der erfindungsgemäßen Verbindungen der Formel (I) vorzugsweise bzw. als insbesondere bevorzugt angegeben wurde und $Q^4$ steht vorzugsweise für Chlor.

Als Beispiele für die Verbindungen der Formel (V) seien genannt:
Benzolsulfonsäurechlorid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonyl-benzolsulfonsäurechlorid sowie (2-Chlor-phenyl)-, (2-Cyano-phenyl)-, (2-Difluormethoxy-, 2-Trifluormethoxy-, 2-(2-Chlorethoxy)-, 2-Methoxycarbonyl-phenyl)- und (2-Trifluormethoxy-phenyl)methansulfonsäurechlorid, 2-Chlor-6-methyl-benzolsulfonsäurechlorid und 2,6-Dichlor-benzolsulfonsäurechlorid.

Die Sulfonsäurehalogenide bzw. -anhydride der Formel (V) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Die weiter als Zwischenprodukte benötigen Heteroazole sind durch die Formel (VI) allgemein definiert. In Formel (VI) haben A, D und E vorzugsweise bzw. insbesondere diejenigen Bedeutungen, die bereits oben im Zusammenhang mit der Beschreibung der erfindungsgemäßen Verbindung der Formel (I) vorzugsweise bzw. als insbesondere bevorzugt für A, D und E angegeben wurden und $Q^5$ steht vorzugsweise für Fluor, Chlor, Brom, oder $C_1$-$C_4$-Alkylsulfonyl, insbesondere für Chlor oder Methylsulfonyl.

Als Beispiele für die Verbindungen der Formel (VI) seien genannt:
2-Chlor-oxazol, 2-Chlor-thiazol, 2-Chlor- und 2-Methylsulfonyl-1,3,4-thiadiazol, 2-Chlor-1,3,4-oxadiazol, 5-Chlor-1,2,4-thiadiazol, 2-Chlor-4-methyl-thiazol, 2,4,5-Trichlor-thiazol, 2-Chlor-5-methyl-thiazol, 2-Chlor- und 2-Methylsulfonyl-5-methyl-1,3,4-thiadiazol, 2-Chlor- und 2-Methylsulfonyl-5-ethyl-1,3,4-thiadiazol, 2-Chlor- und 2-Methylsulfonyl-5-trifluormethyl-1,3,4-thiadiazol, 2-Chlor- und 2-Methylsulfonyl-5-difluormethyl-1,3,4-thiadiazol, 2-Chlor- und 2-Methylsulfonyl-5-dichlormethyl-1,3,4-thiadiazol, 2-Chlor- und 2-Methylsulfonyl-5-methylthio-1,3,4-thiadiazol und 2-Chlor-5-phenyl-1,3,4-thiadiazol.

Die Heteroazole der Formel (VI) sind bekannt und/oder können nach an sich bekannten Verfahren hergestellt werden (vgl. Comprehensive Heterocyclic Chemistry, Vol 6, Pergamon Press 1984).

Die weiter als Zwischenprodukte zu verwendenden Sulfonsäureamide sind durch die Formel (VII) allgemein definiert. In Formel (VII) hat $R^1$ vorzugsweise bzw. insbe sondere diejenige Bedeutung, die oben im Rahmen der Beschreibung der erfindungsgemäßen Verbindungen der Formel (I) vorzugsweise bzw. als insbesondere bevorzugt angegeben wurde.

Als Ausgangsstoffe der Formel (VII) seien beispielsweise genannt:

Benzolsulfonsäureamid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Difluormethoxy-, 2-Trifluormethoxy-, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl-, und 2-Diethylaminocarbonyl-benzolsulfonsäureamid sowie (2-Chlor-phenyl)-, (2-Cyano-phenyl)-, (2-Methoxycarbonyl-phenyl)- und (2-Trifluormethoxy-phenyl)methansulfonsäureamid, 2-Chlor-6-methyl-benzolsulfonsäurechlorid und 2,6-Dichlor-benzolsulfonsäureamid.

Die Sulfonsäureamide der Formel (VII) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Die beim erfindungsgemäßen Verfahren zur Herstellung der neuen Verbindungen der Formel (I) weiter als Ausgangsstoffe zu verwendenden Azine sind durch die Formel (III) allgemein definiert. In Formel (III) haben $R^2$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, X, Y und Z angegeben wurden, und Q steht vorzugsweise für Fluor, Chlor, Brom oder $C_1$-$C_4$-Alkylsulfonyl, insbesondere für Chlor oder Methylsulfonyl.

Als Beispiele für die Verbindungen der Formel (III) seien genannt:

2-Chlor- und 2-Methylsulfonyl-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidin, -4-chlor-6-methyl-pyrimidin, -4-trifluormethyl-6-methoxy-pyrimidin, -4-methoxy-6-difluormethoxy-pyrimidin, -4-methyl-6-difluormethoxy-pyri midin, -4,6-bis-difluormethoxy-pyrimidin, -4-chlor-6-ethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-dimethoxy-pyrimidin sowie -4,6-bis-trifluormethyl-pyrimidin, ferner 2-Chlor-4,6-dimethyl-s-triazin, 2-Chlor-4-methyl-6-methoxy-s-triazin, 2-Chlor-4,6-dimethoxy-s-triazin, 2,4-Dichlor-6-methoxy-s-triazin, 2-Chlor-4-ethyl-6-methoxy-s-triazin und 2-Chlor-4-methyl-6-ethoxy-s-triazin.

Die Azine der Formel (III) sind bekannt und/oder können nach an sich bekannten Verfahren hergestellt werden (vgl. J. Chem. Soc. 1957, 1830, 1833; J. Org. Chem. 26 (1961), 792; US-P 3 308 119 und US-P 4 711 959).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren wird im allgemeinen unter Normaldruck durchgeführt. Es ist jedoch

auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als

Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D);
4-(2,4-Di-chlor-phen-oxy)-but-ter-säu-re (2,4-DB);
2,4-Dichlorphenoxypropionsäure (2,4-DP);
5-(2-Chlor-4-trifluormethyl-phenoxy)-2-nitro-benzoesäure (ACIFLUORFEN);
Chloressigsäure-N-(methoxymethyl)-2,6-diethylanilid (ALACHLOR);
Methyl-6,6-dimethyl-2,4-dioxo-3-[1-(2-propenyloxyamino)butyliden]-cyclohexancarbonsäure (ALLOXYDIM);
2-Chlor-4-ethylamino-6-isopropylamino-1,3,5-triazin (ATRAZIN);
3-Isopropyl-2,1,3-benzothiadiazin-4-on-2,2-dioxid (BENTAZON);
Methyl-5-(2,4-dichlorphenoxy)-2-nitrobenzoat (BIFENOX);
3,5-Dibrom-4-hydroxy-benzonitril (BROMOXYNIL);
N-(Butoxymethyl)-2-chlor-N-(2,6-diethylphenyl)-acetamid (BUTACHLOR);
N,N-Dimethyl-N′-(3-chlor-4-methylphenyl)-harnstoff (CHLORTOLURON);
exo-1-Methyl-4-(1-methylethyl)-2-(2-methylphenyl-methoxy)-7-oxabicyclo-(2,2,1)-heptan (CINMETHYLIN);
3,6-Dichlor-2-pyridincarbonsäure (CLOPYRALID);
2-Chlor-4-ethylamino-6-(3-cyanopropylamino)-1,3,5-triazin (CYANAZIN);
2-[4-(2,4-Dichlorphenoxy)-phenoxy]-propionsäure, deren Methyl- oder deren Ethylester (DICLOFOP);
N,N-Di-n-propyl-Thiocarbamidsäure-S-ethylester (EPTAME);
4-Amino-6-t-butyl-3-ethylthio-1,2,4-triazin-5(4H)-on (ETHIOZIN);
2-{4-[(6-Chlor-2-benzoxazolyl)-oxy]-phenoxy}-propansäure, deren Methyl- oder deren Ethylester (FENOXAPROP);
2-[4-(5-Trifluormethyl-2-pyridyloxy)-phenoxy]-propansäure oder deren Butylester (FLUAZIFOP);
[(4-Amino-3,5-dichlor-6-fluor-2-pyridinyl)-oxy]-essigsäure bzw. deren 1-Methylheptylester (FLUROXYPYR);
5-(2-Chlor-4-trifluormethyl-phenoxy)-N-methylsulfonyl-2-nitrobenzamid (FOMESAFEN);
2-{4-[(3-Chlor-5-(trifluormethyl)-2-pyridinyl)-oxy]-phenoxy}-propansäure bzw. deren Ethylester

(HALOXYFOP);
3,5-Diiod-4-hydroxybenzonitril (IOXYNIL);
N,N-Dimethyl-N'-(4-isopropylphenyl)-harnstoff (ISOPROTURON);
(2-Ethoxy-1-methyl-2-oxo-ethyl)-5-[2-chlor-4-(trifluormethyl)-phenoxy]-2-nitrobenzoat (LACTOFEN);
(2-Methyl-4-chlorphenoxy)-essigsäure (MCPA);
(4-Chlor-2-methylphenoxy)-propionsäure (MCPP);
N-Methyl-2-(1,3-benzthiazol-2-yloxy)-acetanilid (MEFENACET);
2-Chlor-N-(2,6-dimethylphenyl)-N-[(1H)-pyrazol-1-yl-methyl]-acetamid (METAZACHLOR);
2-Ethyl-6-methyl-N-(1-methyl-2-methoxyethyl)-chloracetanilid (METOLACHLOR);
S-Ethyl-N,N-hexamethylen-thiolcarbamat (MOLINATE);
4-(Di-n-propylamino)-3,5-dinitrobenzolsulfonamid (ORYZALIN);
(2-Chlor-4-trifluormethylphenyl)-(3-ethoxy-4-nitro-phenyl)-ether (OXYFLUORFEN);
N-(1-Ethylpropyl)-3,4-dimethyl-2,6-dinitroanilin (PENDIMETHALIN);
0-(6-Chlor-3-phenyl-pyridazin-4-yl)-S-octyl-thiocarbonat (PYRIDATE);
2-[1-(Ethoxamino)-butyliden]-5-(2-ethylthiopropyl)-1,3-cyclohexadion (SETHOXYDIM);
2-Chlor-4,6-bis-(ethylamino)-1,3,5-triazin (SIMAZIN);
2,4-Bis-[N-ethylamino]-6-methylthio-1,3,5-triazin (SIMETRYNE);
4-Ethylamino-2-t-butylamino-6-methylthio-s-triazin (TERBUTRYNE);
S-[(4-Chlorphenyl)-methyl]-N,N-diethyl-thiocarbamat (THIOBENCARB);
N,N-Diisopropyl-S-(2,3,3-trichlorallyl)-thiolcarbamat (TRIALLATE);
2,6-Dinitro-4-trifluormethyl-N,N-dipropylanilin (TRIFLURALIN) und 2-[4-(6-Chlor-chinoxalin-2-yl-oxy)-phenoxy]propionsäure-ethylester (QUIZALOFOPETHYL). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die Angewandte Wirkstoffmenge kann in einem größeren Bereich schwanken. Sie hängt im wesentlichen von der Art des gewünschten Effektes ab. Im allgemeinen liegen die Aufwandmengen zwischen 0,005 und 5 kg Wirkstoff pro Hektar Bodenfläche, vorzugsweise zwischen 0,01 und 3 kg pro ha.

Die Herstellung und die Verwendung der erfindungsgemäßen Wirkstoffe geht aus den nachfolgenden Beispielen hervor.

## Herstellungsbeispiele

### Beispiel 1

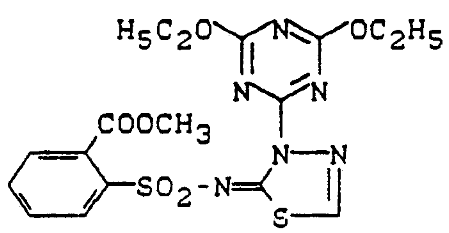

2,99 g (0,01 Mol) 2-(2-Methoxycarbonyl-phenyl-sulfonylamino)-1,3,4-thiadiazol werden in 100 ml Tetrahydrofuran gelöst und bei 25°C mit 1,12 g (0,01 Mol) Kalium-tert-butylat versetzt. Nach 30 Minuten Rühren werden 2,24 g (0,011 Mol) 2-Chlor-4,6-diethoxy-s-triazin dazu gegeben und das Reaktionsgemisch wird 20

Stunden unter Rückfluß zum Sieden erhitzt. Dann wird im Wasserstrahlvakuum eingeengt, der Rückstand mit Methylenchlorid/Wasser geschüttelt, die organische Phase abgetrennt, mit Natriumsulfat getrocknet und filtriert. Vom Filtrat wird das Lösungsmittel im Wasserstrahlvakuum abdestilliert, der Rückstand mit Diethylether verrührt und das hierbei kristallin angefallene Produkt durch Absaugen isoliert.

Man erhält 1,4 g (30 % der Theorie) 2-(2-Methoxycarbonyl-phenylsulfonylimino)-3-(4,6-diethoxy-triazin-2-yl)-2,3-dihydro-1,3,4-thiadiazol vom Schmelzpunkt 97° C.

Analog Beispiel 1 und entsprechend der allgemeinen Beschreibung des erfindungsgemäßen Verfahrens können die in der nachstehenden Tabelle 2 aufgeführten Verbindungen der Formel (I) hergestellt werden.

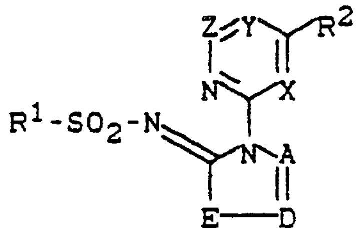

(I)

Tabelle 2

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 2 | N | CH | S | 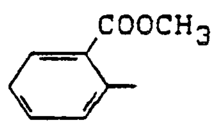 | $OCH_3$ | N | N | $C-OCH_3$ | 103 |
| 3 | CH | CH | S | 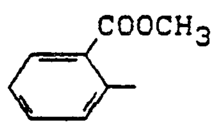 | $OCH_3$ | N | N | $C-OCH_3$ | 153 |
| 4 | N | CH | S | 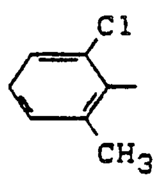 | $OCH_3$ | N | N | $C-OCH_3$ | 157 |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 5 | N | CH | S | 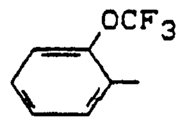 | $OCH_3$ | N | N | $C-OCH_3$ | 159 |
| 6 | N | $C-CH_3$ | S | 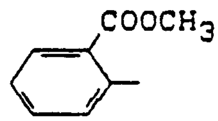 | $OCH_3$ | N | N | $C-OCH_3$ | 118 |
| 7 | N | $C-CH_3$ | S | 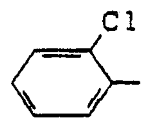 | $OCH_3$ | N | N | $C-OCH_3$ | 101 |
| 8 | N | $C-CH_3$ | S | 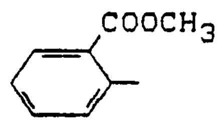 | $OC_2H_5$ | N | N | $C-OC_2H_5$ | 120 |
| 9 | N | $C-CH_3$ | S | 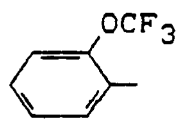 | $OCH_3$ | N | N | $C-OCH_3$ | 76 |
| 10 | N | $C-CH_3$ | S | 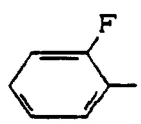 | $OCH_3$ | N | N | $C-OCH_3$ | 151 |
| 11 | N | $C-CH_3$ | S | 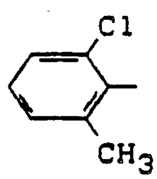 | $OCH_3$ | N | N | $C-OCH_3$ | 97 |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 12 | N | CH | S | 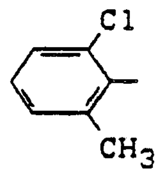 | $OC_2H_5$ | N | N | $C-OC_2H_5$ | 133 |
| 13 | N | $C-CH_3$ | S | 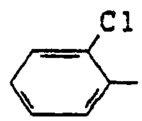 | $OC_2H_5$ | N | N | $C-OC_2H_5$ | 121 |
| 14 | N | $C-CH_3$ | S | 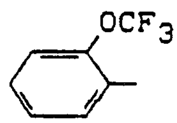 | $OC_2H_5$ | N | N | $C-OC_2H_5$ | 119 |
| 15 | N | $C-CH_3$ | S | 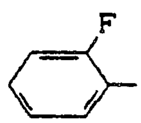 | $OC_2H_5$ | N | N | $C-OC_2H_5$ | 117 |
| 16 | N | CH | S | 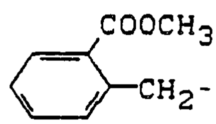 | $OCH_3$ | N | N | $C-OCH_3$ | |
| 17 | N | CH | S | 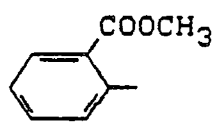 | $CH_3$ | N | CH | $C-CH_3$ | |
| 18 | N | CH | S | 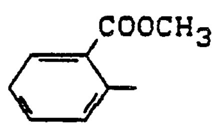 | $CH_3$ | N | CH | $C-OCH_3$ | 142 |
| 19 | N | CH | S | 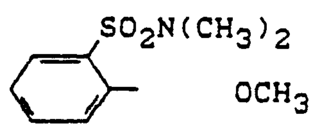 | $OCH_3$ | N | N | $C-OCH_3$ | 187 |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 20 | N | CH | S | 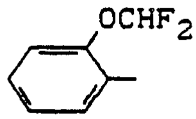 | $OCH_3$ | N | N | $C-OCH_3$ | 162 |
| 21 | CH | CH | S | 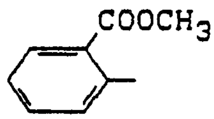 | $CH_3$ | N | CH | $C-OCH_3$ | |
| 22 | CH | CH | S | 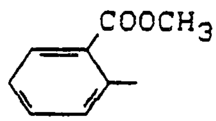 | $OC_2H_5$ | N | N | $C-OC_2H_5$ | |
| 23 | N | $C-CH_3$ | O | 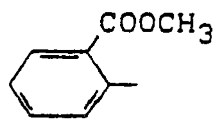 | $OC_2H_5$ | N | N | $C-OC_2H_5$ | |
| 24 | CH | CH | S | 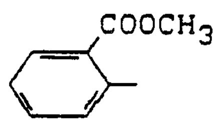 | $CH_3$ | N | N | $C-OCH_3$ | |
| 25 | CH | CH | S | 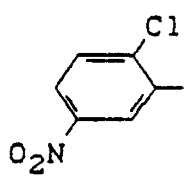 | $CH_3$ | N | CH | $C-CH_3$ | |
| 26 | CH | CH | S | 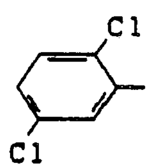 | $CH_3$ | N | CH | $C-CH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 27 | CH | CH | S | 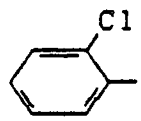 | $OCH_3$ | N | CH | $C-OCH_3$ | |
| 28 | CH | CH | S | 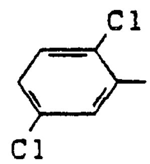 | $OCH_3$ | N | CH | $C-OCH_3$ | |
| 29 | CH | CH | S | 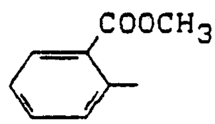 | $CH_3$ | N | CH | $C-CH_3$ | |
| 30 | CH | CH | S | 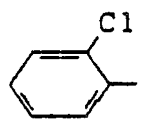 | $CH_3$ | N | CH | $C-OCH_3$ | |
| 31 | CH | CH | S | 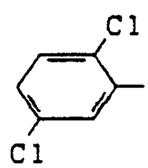 | $CH_3$ | N | CH | $C-OCH_3$ | |
| 32 | CH | CH | S | 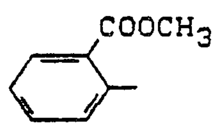 | $OCH_3$ | N | CH | $C-OCH_3$ | |
| 33 | N | CH | S | 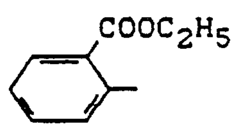 | $CH_3$ | N | CH | $C-OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 34 | CH | CH | S | 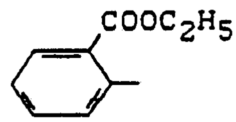 | $OCH_3$ | N | N | $C-OCH_3$ | |
| 35 | CH | CH | S | 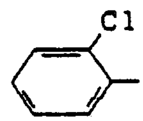 | $OCH_3$ | N | N | $C-OCH_3$ | |
| 36 | N | CH | S | 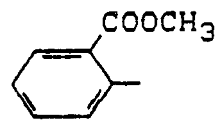 | $OCH_3$ | N | CH | $C-OCH_3$ | |
| 37 | N | CH | S | 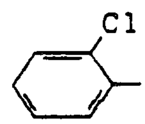 | $OCH_3$ | N | CH | $C-OCH_3$ | |
| 38 | N | CH | S | 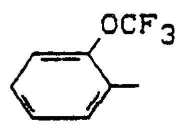 | $CH_3$ | N | CH | $C-CH_3$ | |
| 39 | CH | CH | S | 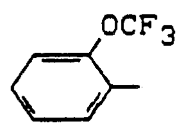 | $CH_3$ | N | CH | $C-CH_3$ | |
| 40 | CH | CH | S | 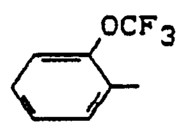 | $CH_3$ | N | CH | $C-OCH_3$ | |
| 41 | N | CH | S | 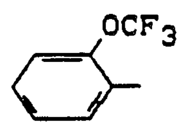 | $CH_3$ | N | CH | $C-OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 42 | N | CH | S | 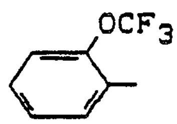 | $OCH_3$ | N | CH | $C-OCH_3$ | |
| 43 | CH | CH | S | 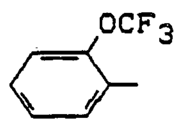 | $OCH_3$ | N | CH | $C-OCH_3$ | |
| 44 | CH | CH | S | 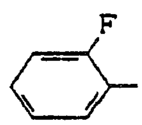 | $OCH_3$ | N | CH | $C-OCH_3$ | |
| 45 | N | CH | S | 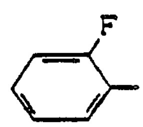 | $CH_3$ | N | CH | $C-CH_3$ | |
| 46 | CH | CH | S | 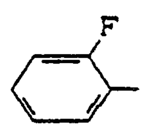 | $CH_3$ | N | CH | $C-CH_3$ | |
| 47 | N | CH | S | 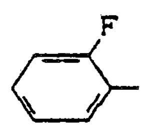 | $OCH_3$ | N | CH | $C-OCH_3$ | |
| 48 | N | CH | S | 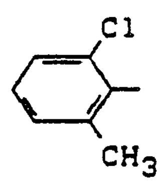 | $CH_3$ | N | CH | $C-CH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 49 | N | CH | S | 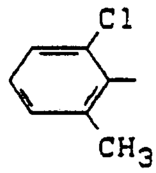 | $CH_3$ | N | CH | $C-OCH_3$ | |
| 50 | N | $C-CH_3$ | S | 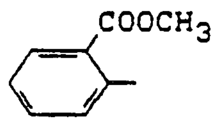 | $CH_3$ | N | CH | $C-CH_3$ | |
| 51 | N | $C-CH_3$ | S | 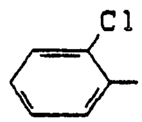 | $CH_3$ | N | CH | $C-CH_3$ | |
| 52 | N | $C-CH_3$ | S | 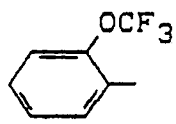 | $CH_3$ | N | CH | $C-CH_3$ | |
| 53 | CH | CH | S | 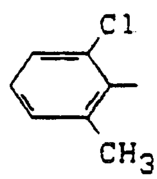 | $CH_3$ | N | CH | $C-OCH_3$ | |
| 54 | CH | CH | S | 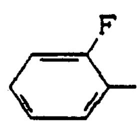 | $CH_3$ | N | CH | $C-OCH_3$ | |
| 55 | N | $C-CH_3$ | S | 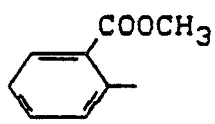 | $OCH_3$ | N | CH | $C-OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 56 | N | $C-CH_3$ | S | 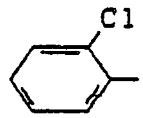 | $CH_3$ | N | CH | $C-OCH_3$ | |
| 57 | N | $C-CH_3$ | S | 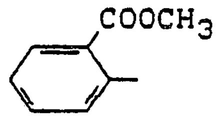 | $CH_3$ | N | CH | $C-OCH_3$ | |
| 58 | N | $C-CH_3$ | S | 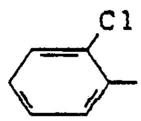 | $CH_3$ | N | CH | $C-OCH_3$ | |
| 59 | N | CH | S | 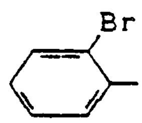 | $CH_3$ | N | CH | $C-OCH_3$ | |
| 60 | N | CH | S | 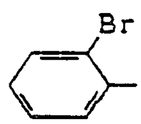 | $OCH_3$ | N | CH | $C-OCH_3$ | |
| 61 | N | $C-C_2H_5$ | S | 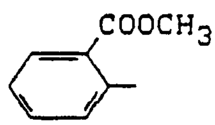 | $CH_3$ | N | CH | $C-OCH_3$ | |
| 62 | N | $C-C_2H_5$ | S | 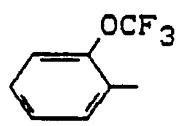 | $CH_3$ | N | CH | $C-OCH_3$ | |
| 63 | N | CH | S | 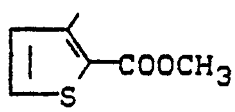 | $CH_3$ | N | CH | $C-CH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 64 | N | CH | S | 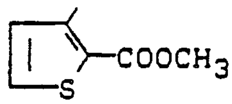 | $CH_3$ | N | CH | C-$OCH_3$ | |
| 65 | N | CH | S | 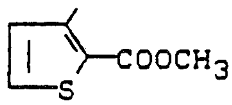 | $OCH_3$ | N | CH | C-$OCH_3$ | |
| 66 | N | CH | S | 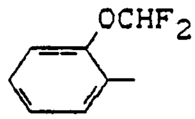 | $CH_3$ | N | CH | C-$OCH_3$ | |
| 67 | N | CH | S | 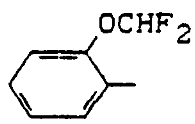 | $CH_3$ | N | CH | C-$OCH_3$ | |
| 68 | N | CH | S | 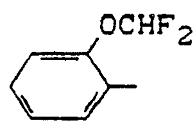 | $OCH_3$ | N | CH | C-$OCH_3$ | |
| 69 | N | C-$SCH_3$ | S | 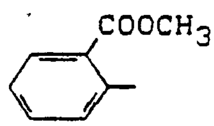 | $CH_3$ | N | CH | C-$CH_3$ | |
| 70 | N | CH | S | 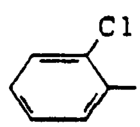 | $CH_3$ | N | CH | C-$OCH_3$ | |
| 71 | N | CH | S | 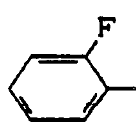 | $CH_3$ | N | CH | C-$OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 72 | N | CH | S | 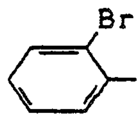 | $CH_3$ | N | CH | $C-CH_3$ | |
| 73 | N | CH | S | 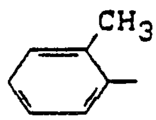 | $CH_3$ | N | CH | $C-OCH_3$ | |
| 74 | N | CH | S | 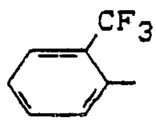 | $CH_3$ | N | CH | $C-CH_3$ | |
| 75 | N | CH | S | 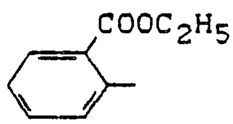 | $CH_3$ | N | CH | $C-CH_3$ | |
| 76 | N | CH | S | 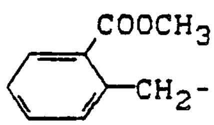 | $OCH_3$ | N | CH | $C-OCH_3$ | |
| 77 | N | CH | S | 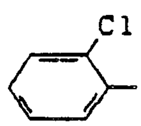 | $CH_3$ | N | N | $C-OCH_3$ | |
| 78 | N | CH | S | 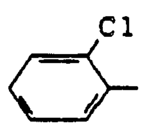 | $OCH_3$ | N | N | $C-OCH_3$ | |
| 79 | N | CH | S | 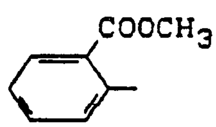 | $CH_3$ | N | N | $C-OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 80 | N | CH | S | 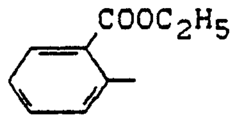 | $OCH_3$ | N | N | $C-OCH_3$ | |
| 81 | N | CH | S | 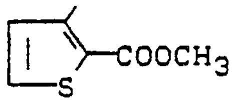 | $CH_3$ | N | N | $C-OCH_3$ | |
| 82 | N | CH | S | 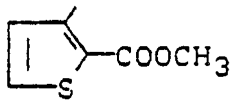 | $OCH_3$ | N | N | $C-OCH_3$ | |
| 83 | N | $C-SCH_3$ | S | 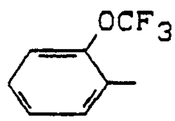 | $OCH_3$ | N | N | $C-OCH_3$ | |
| 84 | CH | CH | S | 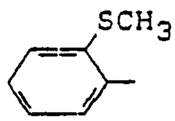 | $OCH_3$ | N | CH | $C-OCH_3$ | |
| 85 | N | CH | S | 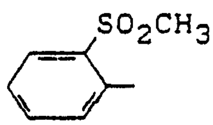 | $OCH_3$ | N | N | $C-OCH_3$ | |
| 86 | N | CH | S | 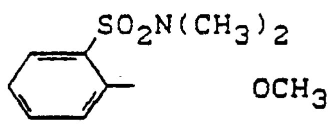 | $OCH_3$ | N | CH | $C-OCH_3$ | |
| 87 | N | CH | S | 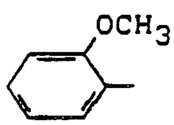 | $CH_3$ | N | CH | $C-CH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 88 | N | CH | S | 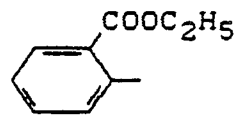 | $OCH_3$ | N | CH | C-Cl | |
| 89 | N | CH | S | 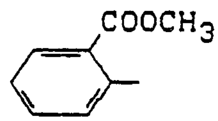 | $OCHF_2$ | N | CH | $C\text{-}OCHF_2$ | |
| 90 | N | CH | S | 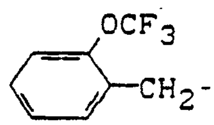 | $OCH_3$ | N | CH | $C\text{-}OCH_3$ | |
| 91 | N | CH | S | 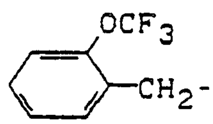 | $OCH_3$ | N | N | $C\text{-}OCH_3$ | |
| 92 | N | CH | S | 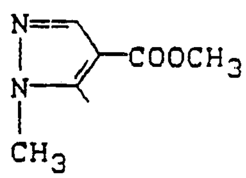 | $OCH_3$ | N | CH | $C\text{-}OCH_3$ | |
| 93 | N | CH | S | 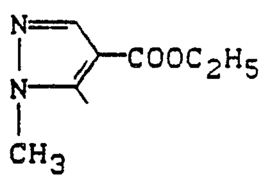 | $OCH_3$ | N | CH | $C\text{-}OCH_3$ | |
| 94 | N | CH | S | 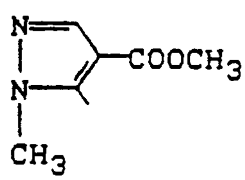 | $OCH_3$ | N | N | $C\text{-}OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 95 | N | CH | S | 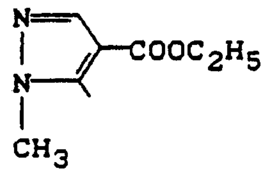 | $OCH_3$ | N | N | $C-OCH_3$ | |
| 96 | N | CH | S | 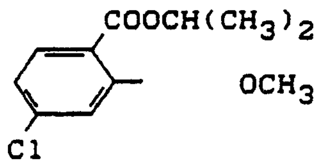 | $OCH_3$ | N | N | $C-OCH_3$ | |
| 97 | N | CH | S | 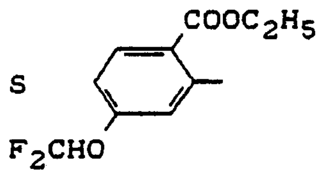 | $OCH_3$ | N | CH | $C-OCH_3$ | |
| 98 | N | CH | S | 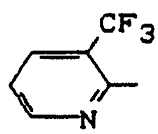 | $OCH_3$ | N | CH | $C-OCH_3$ | |
| 99 | N | CH | S | 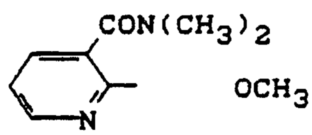 | $OCH_3$ | N | CH | $C-OCH_3$ | |
| 100 | N | CH | S | 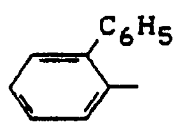 | $OCH_3$ | N | CH | $C-OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 101 | CH | CH | S | 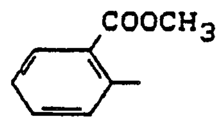 | $CH_3$ | N | CH | $C-CH_3$ | |
| 102 | N | CH | S | 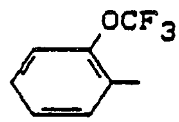 | $OC_2H_5$ | N | N | $C-OC_2H_5$ | |
| 103 | N | CH | S | 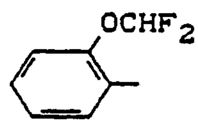 | $OC_2H_5$ | N | N | $C-OC_2H_5$ | 132 |
| 104 | N | CH | S | 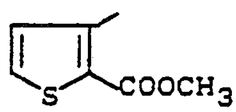 | $OCH_3$ | N | N | $C-OCH_3$ | 128 |
| 105 | N | CH | S | 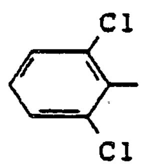 | $OCH_3$ | N | N | $C-OCH_3$ | 165 |
| 106 | N | CH | S | 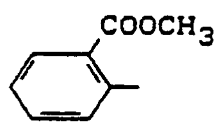 | $OC_2H_5$ | N | N | $C-OCH_3$ | 98 |
| 107 | N | CH | S | 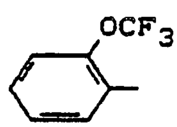 | $OC_2H_5$ | N | N | $C-OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 108 | N | CH | S | 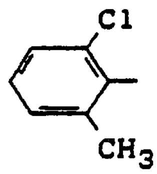 | $OC_2H_5$ | N | N | $C\text{-}OCH_3$ | 137 |
| 109 | N | CH | S | 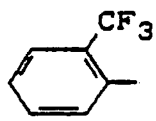 | $OCH_3$ | N | N | $C\text{-}OCH_3$ | |
| 110 | N | CH | S | 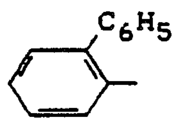 | $OCH_3$ | N | N | $C\text{-}OCH_3$ | |
| 111 | N | CH | S | 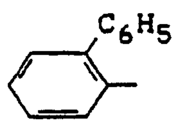 | $OC_2H_5$ | N | N | $C\text{-}OCH_3$ | |
| 112 | N | CH | S | 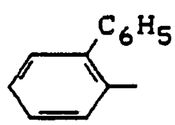 | $OC_2H_5$ | N | N | $C\text{-}OC_2H_5$ | |
| 113 | N | CH | S | 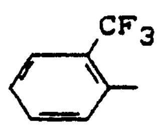 | $OC_2H_5$ | N | N | $C\text{-}OCH_3$ | |
| 114 | N | CH | S | 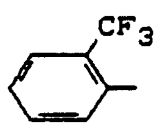 | $OC_2H_5$ | N | N | $C\text{-}OC_2H_5$ | |
| 115 | N | CH | S | 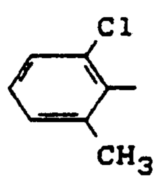 | $CH_3$ | N | N | $C\text{-}CH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 116 | N | CH | S | 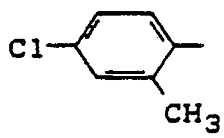 | $OCH_3$ | N | N | C-$OCH_3$ | |
| 117 | CH | CH | S | 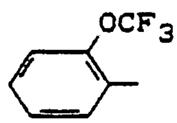 | $OCH_3$ | N | N | C-$OCH_3$ | |
| 118 | N | CH | S | 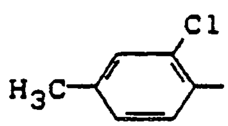 | $OCH_3$ | N | N | C-$OCH_3$ | |
| 119 | CH | CH | S | 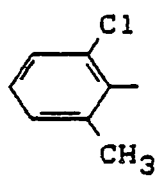 | $OCH_3$ | N | N | C-$OCH_3$ | |
| 120 | CH | CH | S | 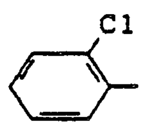 | $OCH_3$ | N | N | C-$OCH_3$ | |
| 121 | CH | CH | S | 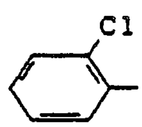 | $CH_3$ | N | CH | C-$OCH_3$ | |
| 122 | CH | CH | S | 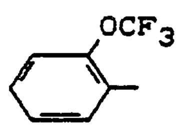 | $CH_3$ | N | CH | C-$OCH_3$ | |
| 123 | CH | CH | S | 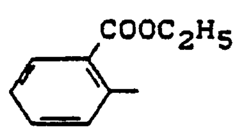 | $CH_3$ | N | CH | C-$OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 124 | CH | CH | S | 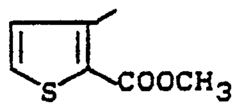 | $CH_3$ | N | CH | $C-OCH_3$ | |
| 125 | CH | CH | S | 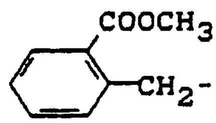 | $OC_2H_5$ | N | N | $C-OC_2H_5$ | |
| 126 | CH | CH | S | 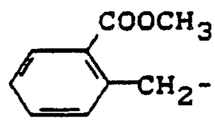 | $OC_2H_5$ | N | N | $C-OCH_3$ | |
| 127 | CH | CH | S | 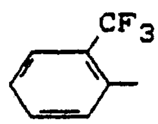 | $CH_3$ | N | CH | $C-OCH_3$ | |
| 128 | CH | CH | S | 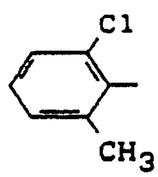 | $OC_2H_5$ | N | N | $C-OCH_3$ | |
| 129 | CH | CH | S | 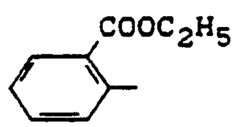 | $OCH_3$ | N | N | $C-OCH_3$ | |
| 130 | CH | CH | S | 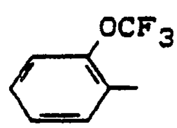 | $OC_2H_5$ | N | N | $C-OCH_3$ | |
| 131 | CH | CH | S | 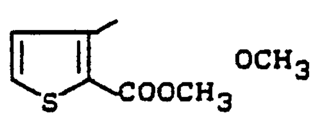 | $OCH_3$ | N | N | $C-OCH_3$ | |

Tabelle 2 - Fortsetzung

| Bsp. Nr. | A | D | E | $R^1$ | $R^2$ | X | Y | Z | Schmelzpunkt °C |
|---|---|---|---|---|---|---|---|---|---|
| 132 | CH | CH | S | 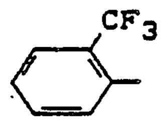 | $OC_2H_5$ | N | N | $C-OCH_3$ | |
| 133 | CH | CH | S | 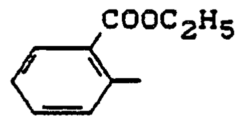 | $OC_2H_5$ | N | N | $C-OCH_3$ | |
| 134 | CH | CH | S | 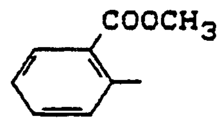 | $OCH_3$ | N | N | $C-OCH_3$ | |
| 135 | CH | CH | S | 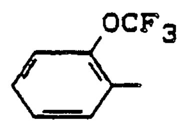 | $CH_3$ | N | CH | $C-CH_3$ | 175 |
| 136 | CH | CH | S | 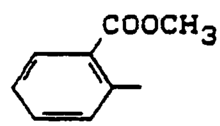 | $CH_3$ | N | CH | CH | |

Ausgangsstoffe der Formel (II)

Beispiel (II-1)

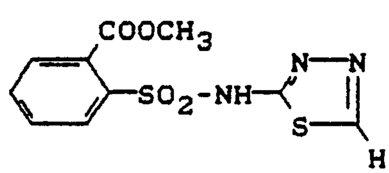

Eine Mischung aus 10,1 g (0,10 Mol) 2-Amino-1,3,4-thiadiazol und 50 ml Pyridin wird auf - 10 °C abgekühlt und bei dieser Temperatur unter Rühren portionsweise mit 31,2 g (0,12 Mol) 2-Methoxycarbonyl-benzolsulfonsäurechlorid (90 %ig) versetzt. Dann wird das Reaktionsgemisch noch 2 Stunden bei - 10 °C und weitere 20 Stunden bei + 25 °C gerührt und anschließend im Wasserstrahlvakuum eingeengt. Der Rückstand wird in Dichlormethan aufgenommen, die Lösung wird mit Wasser gewaschen, mit Magnesiumsulfat getrocknet und filtriert. Vom Filtrat wird das Lösungsmittel im Wasserstrahlvakuum abdestilliert. Der Rückstand wird in 5 %iger wässriger Natronlauge gelöst, das Produkt durch Zugabe von konzentrierter Salzsäure ausgefällt und durch Absaugen isoliert.

Man erhält 21,5 g (71 % der Theorie) 2-(2-Methoxycarbonyl-phenylsulfonylamino)-1,3,4-thiadiazol vom Schmelzpunkt 96 °C.

Beispiel (II-2)

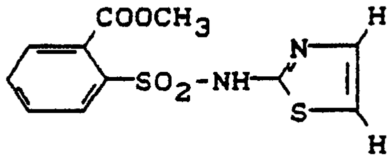

Eine Mischung aus 10,0 g (0,10 Mol) 2-Amino-thiazol und 50 ml Pyridin wird auf -10° C abgekühlt und bei dieser Temperatur unter Rühren portionsweise mit 62,4 g (0,24 Mol) 2-Methoxycarbonyl-benzolsulfonsäurechlorid (90 %ig) versetzt. Dann wird das Reaktionsgemisch noch 2 Stunden bei -10° C und weitere 20 Stunden bei +25° C gerührt und anschließend im Wasserstrahlvakuum eingeengt. Der Rückstand wird in Dichlormethan aufgenommen, die Lösung wird mit Wasser gewaschen, mit Natriumsulfat getrocknet und filtriert. Vom Filtrat wird das Lösungsmittel im Wasserstrahlvakuum abdestilliert, der Rückstand mit Ethanol zur Kristallisation gebracht und das Produkt durch Absaugen isoliert.

Man erhält 25,8 g (56 % der Theorie) 2-(Bis-(2-methoxycarbonyl-phenylsulfonyl)-amino)-thiazol vom Schmelzpunkt 167° C.

22,9 g (0,05 Mol) 2-(Bis-(2-methoxycarbonyl-phenylsulfonyl)-amino)-thiazol werden in 100 ml Ethanol aufgenommen und nach Zugabe von 100 ml konzentriertem wässrigem Ammoniak wird das Gemisch 30 Minuten unter Rückfluß zu Sieden erhitzt. Dann wird im Wasserstrahlvakuum eingeengt, der Rückstand in 200 ml Wasser aufgenommen und durch Zugabe von konzentrierter Salzsäure auf pH 6 eingestellt. Das hierbei kristallin angefallene Produkt wird durch Absaugen isoliert.

Man erhält 9,2 g (61 % der Theorie) 2-(2-Methoxycarbonyl-phenylsulfonylamino)-thiazol vom Schmelzpunkt 196° C.

Analog Beispiel (II-1) oder (II-2) können die in der nachstehenden Tabelle 3 aufgeführten Verbindungen der Formel (II) hergestellt werden.

Tabelle 3

Beispiele für die Verbindungen der Formel (II)

| Bsp. Nr. | A | D | E | $R^1$ | Schmelzpunkt (°C) |
|---|---|---|---|---|---|
| II-3 | N | CH | S | 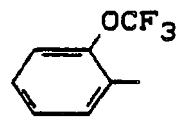 | 160 |
| II-4 | N | $C-CH_3$ | S | 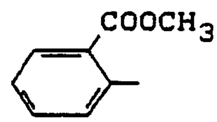 | 143 |
| II-5 | N | CH | S | 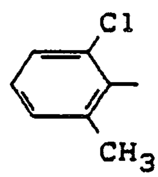 | 132 |
| II-6 | N | $C-CH_3$ | S | 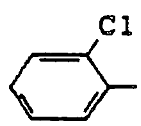 | 185 |
| II-7 | N | $C-CH_3$ | S | 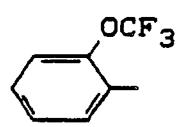 | 135 |
| II-8 | N | $C-CH_3$ | S | 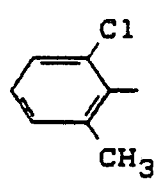 | 180 |
| II-9 | N | $C-CH_3$ | S | 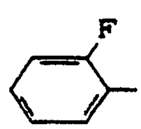 | 154 |

Tabelle 3 - Fortsetzung

Beispiele für die Verbindungen der Formel (II)

| Bsp. Nr. | A | D | E | $R^1$ | Schmelzpunkt (°C) |
|---|---|---|---|---|---|
| II-10 | N | C-$CF_3$ | S | 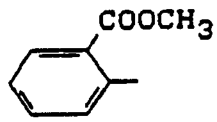 | 65 |
| II-11 | N | CH | S | 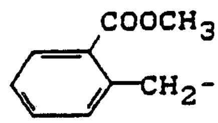 | |
| II-12 | N | CH | S | 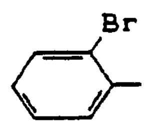 | 241 |
| II-13 | C-Cl | N | O | 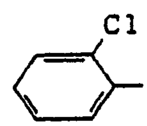 | |

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 1 und 5.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 2 und 5.

## Ansprüche

1. Sulfonylimino-azinylheteroazole der allgemeinen Formel (I)

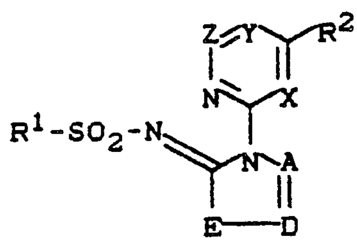

(I)

in welcher
A für Stickstoff oder die Gruppierung C-$A^1$ steht, worin
$A^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,
D für Stickstoff oder die Gruppierung C-$D^1$ steht, worin
$D^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,
(mit der Maßgabe, daß nicht A und D beide gleichzeitig für Stickstoff stehen),
E für Sauerstoff oder Schwefel steht,
$R^1$ für jeweils gegebenenfalls substituiertes Alkyl, Aralkyl, Aryl oder Heteroaryl steht,
$R^2$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht,
X für Stickstoff oder eine CH-Gruppierung steht,
Y für Stickstoff oder die Gruppierung C-$R^3$ steht, worin
$R^3$ für Wasserstoff, Halogen, Cyano, Alkyl, Formyl, Alkylcarbonyl oder Alkoxycarbonyl steht, und
Z für Stickstoff oder die Gruppierung C-$R^4$ steht, worin
$R^4$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht.

2. Verfahren zur Herstellung von Sulfonylimino-azinylheteroazolen der Formel (I) gemäß Anspruch 1, dadurch gekennzeichnet, daß man Sulfonylaminoheteroazole der allgemeinen Formel (II)

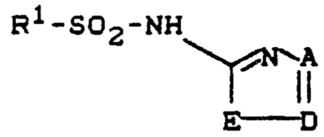

(II)

in welcher
A, D, E und $R^1$ die in Anspruch 1 angegebenen Bedeutungen haben,
mit Azinen der allgemeinen Formel (III)

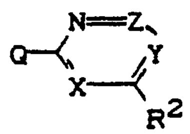

(III)

in welcher
$R^2$, X, Y und Z die in Anspruch 1 angegebenen Bedeutungen haben und
Q für Halogen oder Alkylsulfonyl steht,
gegebenenfalls in Gegenwart eines Säureakzeptors und gegebenenfalls in Gegenwart eines Verdünnungsmittels umsetzt.

3. Herbizide Mittel, gekennzeichnet durch einen Gehalt an mindestens einem Sulfonylimino-azinylheteroazol der Formel (I) gemäß Anspruch 1.

4. Verfahren zur Bekämpfung von Unkräutern, dadurch gekennzeichnet, daß man Sulfonylimino-azinylheteroazole der Formel (I) gemäß Anspruch 1 auf die Unkräuter und/oder ihren Lebensraum einwirken läßt.

5. Verwendung von Sulfonylimino-azinylheteroazolen der Formel (I) gemäß Anspruch 1 zur Bekämpfung von Unkräutern.

6. Verfahren zur Herstellung von herbiziden Mitteln, dadurch gekennzeichnet, daß man Sulfonyliminoazinylheteroazole der Formel (I) gemäß Anspruch 1 mit Streckmitteln und/oder oberflächenaktiven Mitteln vermischt.

7. Sulfonylamino-heteroazole der allgemeinen Formel (II)

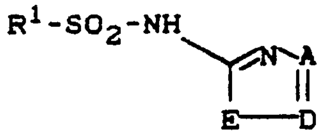

(II)

in welcher
A für Stickstoff oder die Gruppierung C-$A^1$ steht, worin
$A^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,
D für Stickstoff oder die Gruppierung C-$D^1$ steht, worin
$D^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,
(mit der Maßgabe, daß nicht A und D beide gleichzeitig für Stickstoff stehen),
E für Sauerstoff oder Schwefel steht und
$R^1$ für jeweils gegebenenfalls substituiertes Alkyl, Aralkyl, Aryl oder Heteroaryl steht.

Europäisches Patentamt

# EUROPÄISCHER RECHERCHENBERICHT

Nummer der Anmeldung

EP 90102636.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | CHEMICAL ABSTRACTS; Band 100, Nr. 23, 4. Juni 1984, Columbus, Ohio, USA HOSAMNE, RAMACHANDRA S. et al. "Reagents for bioorganic synthesis: preparation, properties and reactions of ethyl and methyl N-(cyanomethyl)methanimidates" Seite 575, Spalte 2, Zusammenfassung Nr. 191 783y & J. Org. Chem. 1984, 49(7), 1212-15 -- | 1 | C 07 D 413/04<br>C 07 D 417/04<br>C 07 D 413/14<br>C 07 D 417/14<br>C 07 D 277/52<br>C 07 D 263/48<br>C 07 D 285/08<br>C 07 D 285/12<br>C 07 D 271/07<br>C 07 D 271/113<br>A 01 N 43/76<br>A 01 N 43/78 |
| A | CHEMICAL ABSTRACTS, Band 100, Nr. 7, 13. Februar 1984, Columbus, Ohio, USA VERCEK, BOJAN et al. "Cyanoamino compounds in synthesis. Synthesis of some heterocycles " Seite 575, Spalte 2, Zusammenfassung Nr. 51 517q & Monatsh. Chem. 1983, 114(6-7), 789-98 -- | 1 | RECHERCHIERTE SACHGEBIETE (Int Cl⁵) |
| A | CHEMICAL ABSTRACTS, Band 72, Nr. 25, 22. Juni 1970, Columbus, Ohio, USA BEDNYAGINA, N.P. et al. "Structure of aromatic and heterocyclic formazans" Seite 270, Spalte 2, Zusammenfassung Nr. 131 663c & Zh. Org. Khim. 1970, 6(3), 619-23 -- | 1 | C 07 D 413/00<br>C 07 D 417/00<br>C 07 D 277/00<br>C 07 D 263/00<br>C 07 D 285/00<br>C 07 D 271/00 |
| | <u>EP - A2 - 0 249 938</u> (NISSAN) * Anspruch 1,11 * | 1,3,6 | |

Der vorliegende Recherchenbericht wurde für alle Patentansprüche erstellt.

| Recherchenort | Abschlußdatum der Recherche | Prüfer |
|---|---|---|
| WIEN | 05-04-1990 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-
EP 90102636.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl ⁵) |
|---|---|---|---|
| | -- | | |
| P,A | EP - A2 - 0 330 959 (BAYER) * Formel I * | 1,3,6, 7 | |
| | -- | | |
| P,A | DE - A1 - 3 737 748 (SCHERING) * Formeln I,XI * | 7 | |
| | -- | | |
| A | US - A - 3 932 437 (DOYLE) * Anspruch I * | 7,6 | |
| | -- | | |
| A | EP - A1 - 0 011 221 (HOFFMANN) * Formel I * | 7,6 | |
| | -- | | |
| A | DD - A - 107 018 (MÜLLER) * Anspruch 1 * | 7 | |
| | ---- | | |

RECHERCHIERTE SACHGEBIETE (Int Cl ⁵)

Der vorliegende Recherchenbericht wurde fur alle Patentanspruche erstellt.

| Recherchenort | Abschlußdatum der Recherche | Prufer |
|---|---|---|
| WIEN | 05-04-1990 | HAMMER |

KATEGORIE DER GENANNTEN DOKUMENTEN

X : von besonderer Bedeutung allein betrachtet
Y : von besonderer Bedeutung in Verbindung mit einer anderen Veroffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veroffentlicht worden ist
D : in der Anmeldung angefuhrtes Dokument
L : aus andern Grunden angeführtes Dokument

& : Mitglied der gleichen Patentfamilie, übereinstimmendes Dokument